Europäisches Patentamt

European Patent Office

Office européen des brevets

(11) Veröffentlichungsnummer: **0 171 671 B1**

(12) **EUROPÄISCHE PATENTSCHRIFT**

(45) Veröffentlichungstag der Patentschrift: **30.09.92**

(21) Anmeldenummer: **85109272.6**

(22) Anmeldetag: **24.07.85**

(51) Int. Cl.⁵: **A61B 19/00**, A41D 13/12

(54) Vorrichtung zur Verhinderung von Infektionen.

(30) Priorität: **01.08.84 DE 3428336**

(43) Veröffentlichungstag der Anmeldung:
**19.02.86 Patentblatt 86/08**

(45) Bekanntmachung des Hinweises auf die
Patenterteilung:
**30.09.92 Patentblatt 92/40**

(84) Benannte Vertragsstaaten:
**AT BE CH DE FR GB IT LI LU NL SE**

(56) Entgegenhaltungen:
**DE-A- 2 908 451**
**FR-A- 2 305 946**
**US-A- 3 706 102**
**US-A- 3 783 451**
**US-A- 3 955 570**

(73) Patentinhaber: **GMT Gesellschaft für medizini-**
**sche Technik mbH**
**Holstenstrasse 2**
**W-2000 Hamburg 50(DE)**

(72) Erfinder: **Engelbrecht, Eckart, Dr. med.**
**Endo-Klinik Holstenstrasse 2**
**W-2000 Hamburg 50(DE)**

(74) Vertreter: **Heldt, Gert, Dr. Dipl.-Ing.**
**Neuer Wall 59 III**
**W-2000 Hamburg 36(DE)**

## Beschreibung

Die Erfindung betrifft eine Vorrichtung zur Verhinderung von Infektionen eines Patienten durch Keime, die während einer Operation von einer dabei anwesenden Person ausgehen, mit einer Absaugeanlage, die die Keime aus ihrem Entstehungsbereich an mindestens einem Körperteil der Person, der während der Operation dem Patienten zugekehrt ist, absaugen kann.

Eine solche Vorrichtung ist in Form einer Gesichtsmaske (US-PS 3 955 570) beschrieben, die im Bereich des Gesichts einer Person eine Gesichtsplatte aufweist. Diese Gesichtsplatte erstreckt sich mit einem Abstand vor dem Gesicht der Person und ist durchsichtig. Diese Gesichtsplatte wird von einer Halterung getragen, die sich auf dem Kopf der Person abstützt. An dieser Halterung sind Belüftungsleitungen beidseits des Gesichts befestigt, durch die einerseits ein Belüftungsstrom geleitet wird und die andererseits als Halterung für die Gesichtsplatte ausgebildet sind. Die Belüftungsleitungen sind mit Öffnungen versehen, durch die Luft aus einem Raum abgesaugt wird, der zwischen dem Gesicht und der Gesichtsplatte vorgesehen ist. Auf diese Weise ist dafür gesorgt, daß die Atemluft der Person durch die Ventilationsöffnungen abgesaugt wird. Darüber hinaus sind in der Gesichtsplatte Belüfungsöffnungen vorgesehen, durch die Luftströme in dem Raum eindringen können, der sich zwischen der Gesichtsplatte und dem Gesicht erstreckt. Die gesamte Maske ist mit Hilfe von Halterungen an dem Kopf der Person befestigt. Ein Abstand zur Gesichtsplatte befindet sich lediglich im Bereich des Gesichtes. In diesem Bereich wird ein Luftstrom durch in die Öffnungen eintretende Luft erzeugt.

Eine solche Gesichtsmaske ist nicht in der Lage, großflächig Keime aus Körperteilen abzusaugen, die sich unterhalb des Kopfes befinden. Gerade die aus diesen großflächigen Körperteilen austretenden Keime sind jedoch leicht in der Lage, Infektionen eines Patienten zu verursachen, der operiert wird und während der Operation diesen Körperteilen unmittelbar benachbart ist. Die von diesen Körperteilen ausgehende Infektionsgefahr ist deswegen besonders groß, weil im Verlaufe der Operation auf relativ großer Fläche vom Körper der an der Operation beteiligten Person Keime austreten. Insbesondere bei körperlich anstrengenden Operationen treten im Bereich des Körpers dieser Person mit deren Schweiß gehäuft Keime aus, die zunächst von der sterilen Kleidung der Person aufgenommen werden. Im Verlauf der Operation sättigt sich die sterile Kleidung zunehmend mit den Ausdünstungen der Person und damit auch mit deren Keimen. Zu einem bestimmten Zeitpunkt ist die Sättigung der Kleidung erreicht, und die Keime treten gehäuft aus, auf der dem Körper abgewandten Oberfläche der inzwischen nicht mehr sterilen Kleidung in Richtung auf das offene Operationsfeld, das sich zu diesem Zeitpunkt möglicherweise in seinem für die Aufnahme von Keimen empfindlichsten Stadium befindet. Zu diesem Zeitpunkt treten die zunächst in der sterilen Kleidung zurückgehaltenen Keime plötzlich in einer starken Häufung aus und infizieren möglicherweise auf gefährliche Weise das Operationsfeld.

Aus diesem Grunde ist versucht worden, Operationskleidung aus einem Stoff herzustellen, der eine möglichst hohe Dichtigkeit gegen durchtretende Keime aufweist. Diese Stoffe sind zwar in der Lage die Keime für eine relativ lange Zeit zurückzuhalten. Während dieser Zeit steigt jedoch der Dampfdruck innerhalb der Kleidung in erheblichem Maße an. Dadurch werden die Keime mit umso größerer Kraft in das Gewebe hineingedrückt, bis sie schließlich auch ein sehr dichtes Gewebe durchdringen. Darüber hinaus haben derartige dichte Gewebe den entscheidenden Nachteil, daß sie einerseits die von ihnen umhüllte Person in besonderer Weise in ihren Bewegungen beeinträchtigen. Andererseits fördern sie aufgrund der stark ansteigenden Temperatur die Schweißausscheidung der Person und damit die Gefahr, das Gewebe mit Keimen zu durchsetzen. Die Keime verteilen sich insbesondere bei einem stark transpirierenden Körper über dessen ganze Fläche.

Aufgabe der vorliegenden Erfindung ist es daher, eine Vorrichtung zu schaffen, die einfach in ihrem Aufbau ist und mit deren Hilfe eine zuverlässige Keimabsaugung flächendeckend möglich ist.

Diese Aufgabe wird erfindungsgemäß dadurch gelöst, daß die Vorrichtung einen im wesentlichen luftundurchlässigen Schild aufweist, der den Körperteil der Person unterhalb deren Kopfes mindestens teilweise überdecken kann; daß der Schild mit mindestens einem auf den Körperteil gerichteten Abstandshalter versehen ist, der sich auf der Oberfläche des Körperteiles abstützen kann, so daß im Gebranch der Schild im Abstand zur Oberfläche des Körperteils verläult mit mindestens einem Zwischenraum zwischen Schild und Oberfläche, in dem ein die Keime abführender Absaugstrom verlaufen kann.

Die wesentliche Bedeutung dieser Erfindung liegt darin, daß nunmehr im Bereich der unterhalb des Kopfes liegenden Körperteile ein luftundurchlässiger Schild zur schweißabsondernden Oberfläche des Patienten hin einen Zwischenraum ausbildet, aus dem die keimhaltigen Ausdünstungen der Person abgesaugt werden. Der luftundurchlässige Schild ermöglicht somit vorteilhaft eine großflächige Absaugung der Keime am Ort des Entstehens und dient gleichzeitig als zuverlässige Schranke gegen die Ausbreitung der Keime. Da die von einem Unterdruckerzeuger angesaugte Luft nicht durch den Schild hindurchdringen kann, sondern nur an den Randbereichen des Schildes vorbei streicht, kann die Förderleistung des Unterdruckerzeugers stark verringert werden.

Es werden auch die in den Randbereichen des Schildes, in denen ein Absaugstrom vorbei streicht, vorhandenen Keime mit abgesaugt.

Gemäß einer bevorzugten Ausführungsform der Erfindung verläuft zwischen dem Schild und dem Körperteil eine Mehr - zahl von Absaugströmen. dies hat den Vorteil, daß in verschiedenen Randbereichen des Schildes vorhandene Keime abgesaugt werden können, so daß beispielsweise, wenn es sich dabei um einen Brustschild handelt, Keime aus einem Halsbereich, aus dem Bereich der Achselhöhlen und aus einem Bauchbereich abgesaugt werden können.

Die Vorrichtung hat ferner den Vorteil, daß sie direkt auf der Haut der mit ihr versehenen Person getragen werden kann. Durch die Absaugströme wird die Haut der Person gekühlt, wodurch eine besonders während einer Operation auftretende Schweißabsonderung und eine damit verbundene Freisetzung von Keimen zuverlässig vermieden wird.

Weitere Einzelheiten der Erfindung ergeben sich aus der nachfolgenden ausführlichen Beschreibung und den beigefügten Zeichnungen, in denen eine bevorzugte Ausführungsform der Erfindung beispielsweise veranschaulicht ist.

In den Zeichnungen zeigen:

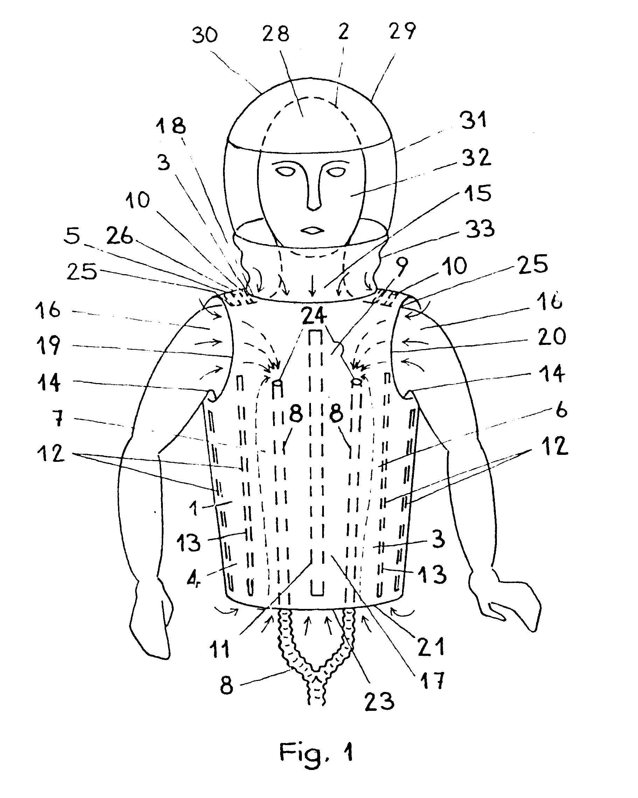

Fig. 1    Eine mit einer Vorrichtung versehene Person in Vorderansicht,

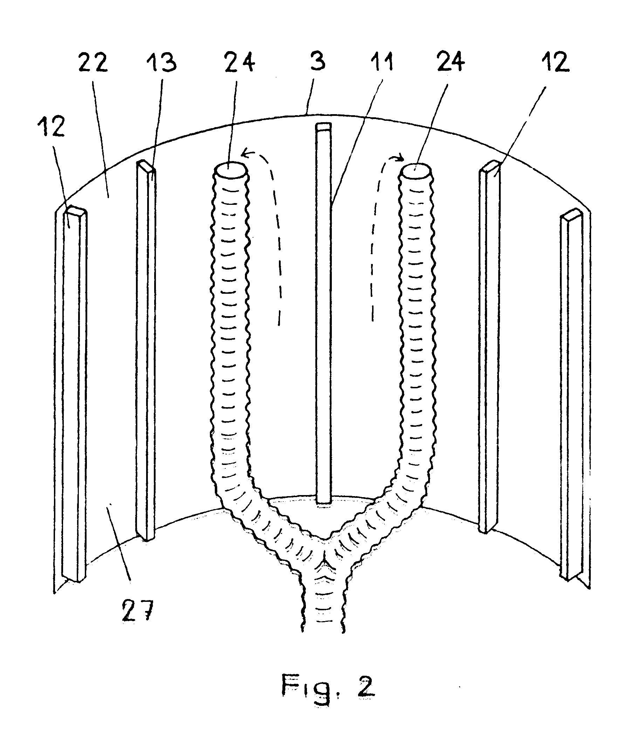

Fig. 2    eine Rückenansicht auf einen Teilausschnitt einer Vorrichtung gemäß Fig. 1,

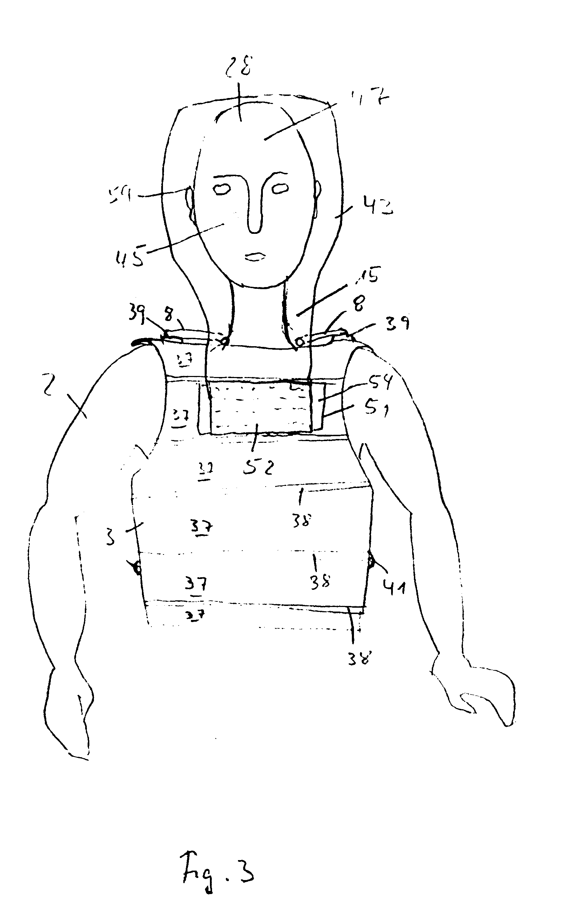

Fig. 3    eine Vorderansicht einer mit einem Kopfschild versehenen Person,

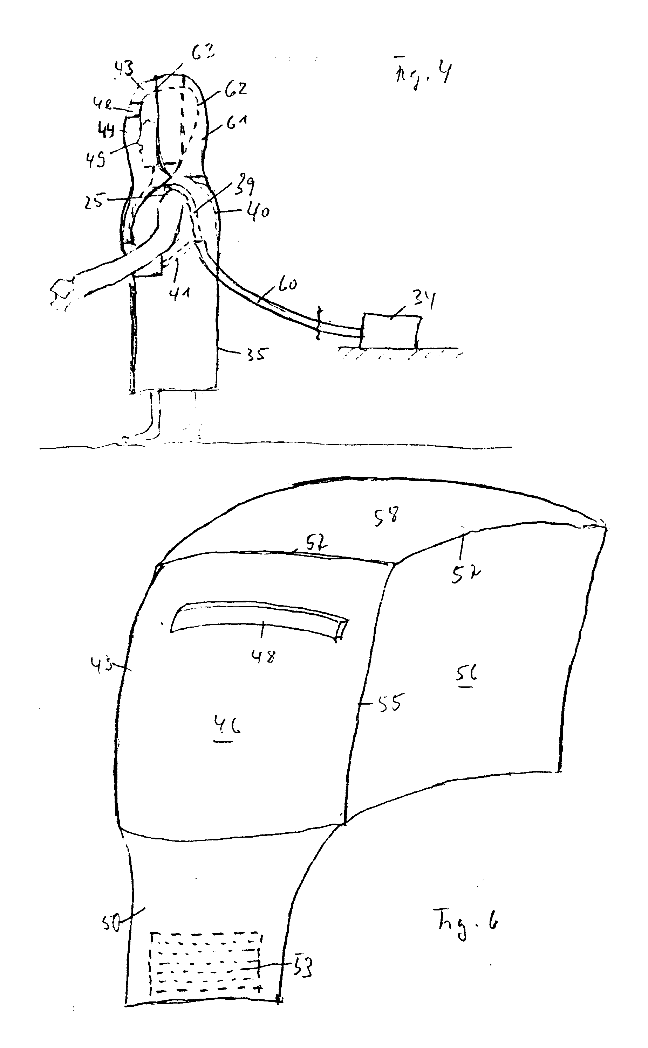

Fig. 4    eine Seitenansicht einer einen Operationskittel oberhalb eines Brustschildes und eines Kopfschildes tragenden Person,

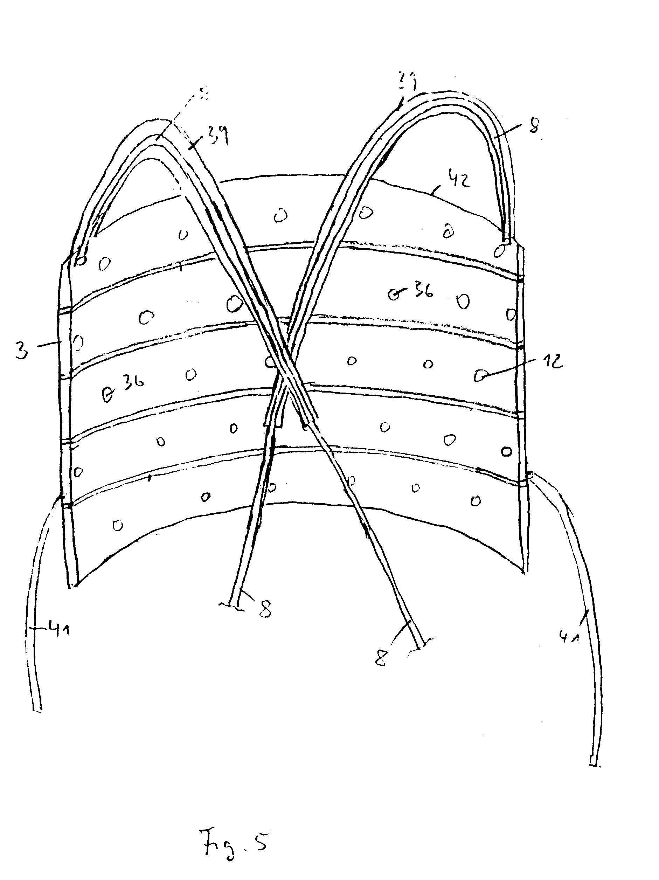

Fig. 5    eine Rückansicht eines Brustschildes und

Fig. 6    eine räumliche Darstellung eines Kopfschildes.

Die Vorrichtung besteht im wesentlichen aus einem einen Körperteil 1 einer Person 2 im wesentlichen überdeckenden Schild 3, der mit einer Oberfläche 4 des Körperteils 1 eine Mehrzahl von Zwischenräumen 5, 6, 7 ausbildet, die über eine flexible Leitung 8 mit einem Unterdruckerzeuger 34 verbunden sind und die von Absaugströmen durchströmt sind.

Der Schild 3 besteht im wesentlichen aus einem aus flexiblem Material gefertigten Brustschild 9, der den aus einer Brust und einer vorderen Schulterpartie der Person 2 bestehenden Körperteil 1 der Person 2 im Wesentlichen überdeckt und im Abstand zu diesem verläuft. Der Schild 3 ist mit auf die Oberfläche 4 des Körperteils 1 ausgerichteten Abstandshaltern 1o, 11, 12 versehen, die sich auf der Oberfläche 4 zumindest teilweise abstützen. Die Abstandshalter 1o, 11, 12 weisen jeweils Abstände zueinander auf. Sie sind als im wesentlichen in gleichen Richtungen verlaufende Abstützleisten 13 ausgebildet, die in ihrem Verlauf einer Form der Oberfläche 4 des Körperteils 1 angepaßt sind. Der Schild 3 weist dem Rücken der Person 2 zugewandte Randbereiche auf, die abdichtend an dem Rücken anliegen. Der Schild 3 umschließt die Achselhöhlen 14 der Person 2 im Wesentlichen und bildet in dem Halsbereich 15, in Bereichen der Oberarmgelenke 16 und in einem Bauchbereich 17 der Person 2 mit der Oberfläche 4 Spalten 18, 19, 2o, 21 aus, die von Absaugströmen durchströmt sind. Auf seiner der Oberfläche 4 zugekehrten Innenseite 22 ist die flexible Leitung 8 befestigt, die an ihrem dem Schild 3 abgewandten Ende mit einem Unterdruckerzeuger verbunden ist und durch die der für die Erzeugung der Absaugströme erforderliche Unterdruck in die Zwischenräume 5, 6, 7 eingebracht wird. Die Leitung[8] ist aus einem dem Bauchbereich 17 zugekehrten unteren Bereich 23 an den Schild 3 herangeführt. Die flexible Leitung 8 ist als Zweigleitung ausgebildet und in mehreren Zwischenräumen 5, 6, 7 sind Saugöffnungen 24 vorgesehen. Die Haupteinströmrichtung der Absaugströme verläuft aus dem Halsbereich 15 in die Saugöffnungen 24.

Der Schild 3 kann mindestens teilweise aus einer flexiblen Folie bestehen. In einem den Schultern 25 der Person 2 zugekehrten Bereich kann der Schild 3 den Schultern 25 soweit angepaßt und versteift sein, daß er die Schultern 25 umgreift und über diese übergehakt werden kann, um den Schild 3 im Bereich der Schultern 25 zu befestigen. Im Rückenbereich der Person 2 ist mindestens ein den Schild 3 festlegender Verschluß vorgesehen, der sich zwischen den einander zugekehrten Randbereichen des Schildes 3 erstreckt.

Der Schild 3 und die Abstandhalter 1o, 11, 12 können entlang ihrer Berührungsflächen mit der Oberfläche 4 mit einem textilen Flächenmaterial beschichtet sein, damit der Kontakt dieser Teile mit der Haut der Person 2 nicht von dieser als unangenehm empfunden wird.

Um eine zu weitgehende Abkühlung der Oberfläche 4 des Körperteiles 1 zu vermeiden, kann die Vorrichtung mit einer von der Körpertemperatur der Person 2 abhängigen Regelung des Luftunterdrukkes zur Steuerung der Menge der angesaugten Luft vorgesehen sein.

Die Vorrichtung kann wie folgt eingesetzt werden:

Eine für eine Mitwirkung bei einer Operation vorgesehene Person 2 überdeckt mit dem Schild 3 ihren dem Patienten während der Operation im wesentlichen zugewandten Körperteil 1, der im wesentlichen aus der Brust und dem Schulterbereich des Oberkörpers der Person 2 besteht. Der Schild 3 wird mit seinen den Schultern 25 zugekehrten Bereichen 26 über die Schultern 25 übergehakt und dort befestigt. Darüber hinaus wird der im Rückenbereich der Person 2 vorgesehene Verschluß verschlossen, so daß am Rücken der Person 2 der Schild 3 abdichtend anliegt. Im Halsbereich 15, im Bereich der Oberarmgelenke 16 und im Bauchbereich 17 bildet der Schild 3 mit der Oberfläche 4 des Körperteils 1 Spalten 18, 19, 2o, 21 aus, durch die Absaugströme in die Zwischenräume 5, 6, 7 einströmen können, wenn der über die flexible Leitung 8 mit dem Schild 3 verbundene Unterdruckerzeuger eingeschaltet wird. Auf diese Weise entstehen im Bereich der Spalten 18, 19, 2o, 21 breitflächig angelegte Absaugströme, durch die in diesen Bereichen vorhandene Keime erfaßt und abgeführt werden können. Da die Vorrichtung unmittelbar auf der Haut getragen wird, werden auch unter dem Schild 3 auf der Oberfläche 4 entstehende Keime durch die Absaugströme abgeführt. Da die Saugöffnungen 24 auf den Halsbereich 15 ausgerichtet sind, werden die wesentlichen Absaugströme im Halsbereich 15 erzielt, so daß insbesondere die vom Kopf der Person 2 ausgehenden Keime zuverlässig abgesaugt werden können.

Ein über der Vorrichtung getragener konventioneller Kittel 35 der Person 2 wird durch die Absaugströme trocken gehalten bzw. beispielsweise nach einer Befeuchtung von außen kurzfristig wieder getrocknet, so daß in soweit die Entstehung von Keimen unterbunden werden kann.

Aufgrund der weitflächigen Absaugung der Keime arbeitet die Vorrichtung sehr leise. Durch die Absaugströme wird die Haut der Person 2 gekühlt, was bei Durchführung einer Operation als sehr angenehm empfunden wird und die mit einer Operation verbundenen Anstrengungen erheblich vermindert.

Gemäß einer bevorzugten Ausführungsform der Erfindung sind die Abstandshalter 1o, 11, 12 als Noppen 36 ausgebildet, die sich auf der Oberfläche 4 abstützen. Hierdurch kann die Entstehung einzelner, voneinander getrennter Zwischenräume 5, 6, 7 vermieden werden, was unter strömungstechnischen Gesichtspunkten erwünscht sein kann. Es ist ferner möglich, die flexible Leitung 8 als Zweigleitung mit so vielen Leitungszweigen auszubilden, daß jeder Zwischenraum 5, 6, 7 mit einer eigenen Saugöffnung 24 versehen ist. Die Saugöffnung 24 kann in einem zentralen Bereich des Schildes 3 angeordnet sein. Sie kann ferner nahe dem Halsbereich 15 angeordnet sein.

Es ist möglich, den Schild 3 am Hals der Person 2 zu haltern. Hierfür kann ein Band vorgesehen sein, das den Hals der Person 2 mindestens teilweise umgibt.

Ferner besteht die Möglichkeit, den Kopf 28 der Person 2 mit einem Helmsystem 29 zu versehen, das aus einer Helmschale 3o und einem durchsichtigen Visier 31 im Gesichtsbereich 32 der Person 2 besteht. Das Helmsystem 29 ist ebenfalls mit dem Unterdruckerzeuger 34 verbunden, so daß Keime, die im Bereich des Kopfes 28 der Person 2 entstehen, ebenfalls abgesaugt werden können. Das Helmsystem 29 kann über eine elastische Manschette 33, die den Halsbereich 15 der Person 2 im wesentlichen umschließt und die bis in die Spalte 18 hineinragt, mit dem Unterdruckerzeuger verbunden sein.

Der Schild 3 kann auch aus flexibel miteinander verbundenen Teilstücken 37 bestehen, die über Schwenkglieder 38 miteinander verbunden sind. Diese Schwenkglieder können aus einem lufttundurchlässigen flexiblen Material, beispielsweise einer Folie, bestehen. Auf diese Weise wird gewährleistet, daß der Schild 3 Beugebewegungen nachvollziehen kann, die die Person 2 ausführt. Auf diese Weise wird von der Person 2 der Schild 3 auch bei der Ausführung komplizierter Bewegungsvorgänge nicht als störend empfunden. Insbesondere ist gewährleistet, daß die Person 2 sich setzen kann, ohne daß zubefürchten ist, daß der Schild 3 sich am Körperteil 1 der Person 2 hochschiebt. Im Bereich der Schwenkglieder 38 können sich auch Abstützleisten erstrecken, die im wesentlichen quer zur Körperlängsachse der Person 2 verlaufen. Falls derartige quer zur Körperlängsachse der Person 2 verlaufende Abstützleisten vorgesehen sind, muß dafür Sorge getragen werden, daß in den Abstützleisten Durchlässe für die im oberen beziehungsweise unteren Teil des Schildes 3 abgesaugte Luft vorgesehen sind.

Um die Luft im oberen Teil des Schildes 3 absaugen zu können, wird die flexible Leitung 8 in den Halsbereich 15 der Person 2 geführt. Dabei können zur Heranführung der Leitung 8 Träger 39 Verwendung finden, die im Bereich der Schultern 25 an dem Schild 3 befestigt sind. Diese Träger 39 weisen eine breite Auflagefläche auf, die sich von der Schulter 25 über einen dem Schild 3 abgewandten Rückenbereich 4o der Person 2 erstreckt. Für einen guten Sitz des Schildes 3 sorgen zwei Träger 39, von denen je einer über je eine Schulter 25 der Person 2 verläuft und sich im Rückenbereich 4o mit dem jeweils anderen Träger 39 kreuzt. Zweckmäßigerweise werden im Kreuzungspunkt der Träger 39 mit diesen Bänder 41 verknüpft, die am Schild 3 in dessen unteren Drittel befestigt sind. Auf diese Weise wird für einen festen Sitz des Schildes 3 gesorgt, ohne daß dieser formschlüssig auf der Oberfläche 4 anliegt. Die Träger 39 sind an einer im Bereich der Schultern 25 verlaufenden Oberkante 42 des Schildes 3 befestigt.

Von diesen Trägern 39 verläuft die Leitung 8 in einen von einem Kopfschild 43 umschlossenen Raum. Dieser Kopfschild 43 erstreckt sich mit einem Gesichtsfeld 44 vor einem Gesicht 45 der Person 2. Dieses Gesichtsfeld 44 kann als eine flache Ebene 46 ausgebildet sein. Es ist jedoch auch möglich, den Kopfschild 43 als eine den Kopf 28 weiträumig umgebende Halbschale auszubilden, die an keiner Stelle auf dem Kopf 28 aufliegt und damit die Bewegungsfreiheit des Kopfes nicht beeinträchtigt. Lediglich im Bereich einer Stirn 47 ist ein Stirnanschlag 48 vorgesehen, gegen den die Stirn 47 anliegt, wenn die Person 2 ihren Kopf 28 vorwärtsbeugt. Auf diese Weise wird vermieden, daß die Person 2 mit ihrer Nase 49 gegen das Gesichtsfeld 44 stößt und dieses dadurch in seiner Durchsichtigkeit beeinträchtigt.

Mit dem Kopfschild 43 ist ein Befestigungsansatz 5o fest verbunden, der sich vom Gesichtsfeld 44 abwärts in Richtung auf das Schild 3 erstreckt. Der Befestigungsansatz 5o überragt das Schild 3 in dessen Brustbereich 51, an dem der Befestigungsansatz 5o befestigt ist. Die Befestigung geschieht mit Hilfe eines Rasterverschlusses, der ohne Schwierigkeiten eine Verbindung des Kopfschildes 43 mit dem Schild 3 zuläßt. Dieser Rasterverschluß kann als ein Klettverschluß 52 ausgebildet sein, dessen einer Teil 53 auf einer dem Schild 3 zugewandten Innenseite des Befestigungsansatzes 5o befestigt ist. Der diesem Teil 53 zugeordnete andere Teil des Klettverschlusses 52 ist auf einer Verstärkung 54 des Schildes 3 vorgesehen, die im Brustbereich 51 auf ein Teilstück 37 aufgebracht ist. Durch Zusammenfügen des Klettverschlusses 52 wird der Kopfschild 43 am Schild 3 befestigt. Diese Befestigung kann durch einen leichten auf den Befestigungsansatz 5o aufzubringenden Druck herbeigeführt werden, ohne daß die diesen Druck aufbringenden Hände sich am Kopfschild 43 mit Keimen kontaminieren können, da der Kopfschild 43 vor seiner Befestigung am Schild 3 sterilisiert wird. Er ist zu diesem Zwecke aus einem für die Sterilisation geeigneten Material hergestellt.

Statt eines Klettverschlusses 52 können auch andere Verschlüsse benutzt werden, um den Kopfschild 43 am Schild 3 zu befestigen. Beispielsweise ist die Verbindung über Druckknöpfe denkbar. Es ist auch möglich, den Befestigungsansatz 5o in eine am Schild 3 vorzusehende Schiebetasche in Richtung der Längsachse der Person 2 von oben einzuschieben. Auch eine Schiebeverbindung in Richtung einer quer zur Längsachse der Person 2 verlaufenden Schiebeverbindung ist möglich.

Das Gesichtsfeld 44 geht im Bereich von Eckkanten 55 in Seitenflächen 56 des Kopfschildes 43 über. Diese Eckkanten 55 können abgerundet sein. Ebenso können Oberkanten 57 abgerundet sein, an denen die Seitenflächen 56 beziehungsweise die flache Ebene 46 des Gesichtsfeldes 44 in eine Deckfläche 58 übergeht. Es ist jedoch auch möglich, den gesamten Kopfschild 43 als Halbschale herzustellen. Der Stirnanschlag 48 erstreckt sich unterhalb der Oberkante 57 des Gesichtsfeldes 44 weitgehend über die gesamte Breite der flachen Ebene 46. Auf diese Weise wird erreicht, daß der Kopf 28 auch bei Bewegungen innerhalb des Kopfschildes 43 zunächst mit dem Stirnanschlag 48 in Berührung kommt, bevor er an das Gesichtsfeld anstößt.

Durch eine strömungsgünstige Einleitung der Leitung 8 in eine von dem Kopfschild 43 einerseits und dem Schild 3 andererseits ausgebildeten Ecke wird eine günstige Absaugung der Keime aus den Spalten 18, 19, 2o, 21 gewährleistet. Diese Absaugung geschieht weitgehend ohne Absauggeräusche, da der Absaugstrom nicht im Bereich von Ohren 59 der Person 2 vorbeigeführt wird.

Eine geringe Geräuschentwicklung der Vorrichtung beruht bei Verwendung eines Kopfschildes 43 auch darauf, daß sich der Kopfschild 43 auf dem Schild 3 abstützt und nicht auf dem Kopf 28 aufliegt. Dadurch wird eine Geräuschübertragung über die Knochenleitung verhindert.

Der Schild 3 wird zunächst von der Person 2 im Brustbereich 51 angelegt. Sodann werden die Träger 39 miteinander und mit den Bändern 41 verknüpft. Daraufhin kann die Leitung 8 über eine entsprechende Verbindungsleitung 6o mit dem Unterdruckerzeuger 34 verbunden werden.

Anschließend wird der Kopfschild 43 über den Klettverschluß 52 mit dem Schild 3 verbunden. Nunmehr kann die Person 2 den Kittel 35 anziehen, der im Brustbereich 51 sowohl den Schild 3 als auch den Befestigungsansatz 5o überdeckt. Eine am Kittel 35 befestigte Kapuze 61 wird über einen vom Kopfschild 43 nicht bedeckten Hinterkopf 62 der Person 2 gezogen, so daß sie mit ihrer dem Gesicht 45 zugewandten Vorderkante 63 über die Deckfläche 58 und die Seitenflächen 56 des Kopfschildes 43 verläuft. Nunmehr kann aus den Spalten 18, 19, 2o, 21 über die Leitung 8 Luft abgesaugt werden. Damit wird verhindert, daß vom Körper der Person 2 Keime den Kittel 35 in Richtung auf ein vor der Person 2 liegendes Operationsfeld durchdringen. Die volle Bewegungsfreiheit der Person 2 bleibt erhalten, da sie weder durch den Kopfschild 43 noch durch den Schild3 beeinträchtigt wird. Der Kopfschild 43 vollzieht sämtliche Bewegungen nach, die die Person 2 mit ihrem vom Schild 3 bedeckten Oberkörper durchführt. Innerhalb des Kopfschildes 43 besitzt der Kopf 28 sämtliche Bewegungsmöglichkeiten, die er benötigt.

Der Schild 3 besitzt außer den Trägern 39 keine Verbindungsmittel, die unmittelbar am Körper der Person 2 anliegen. Eine starre Verbindung zwischen dem Schild 3 und dem Körper der Person 2 besteht nicht.

**Patentansprüche**

1. Vorrichtung zur Verhinderung von Infektionen eines Patienten durch Keime, die während einer Operation von einer dabei anwesenden Person (2) ausgehen, mit einer Absauganlage, die die Keime aus ihrem Entstehungsbereich an mindestens einem Körperteil (1) der Person (2) der während der Operation dem Patienten zugekehrt ist, absaugen kann, wober die Vorrichtung einen im wesentlichen luftundurchlässigen Schild (3) aufweist, und der Schild (3) mit mindestens einem auf den Körperteil (1) gerichteten Abstandshalter (10, 11, 12) versehen ist, der sich auf der Oberfläche (4) des Körperteils (1) abstützen kann, so daß im Gebrauch der Schild (3) im Abstand zur Oberfläche (4) des Körperteils (1) verläuft mit mindestens einem Zwischenraum (5, 6, 7) Zwischen Schild und Oberfläche, in dem ein die Keime abführender Absaugstrom verlaufen kann, dadurch gekennzeichnet, daß der Schild den Körperteil (1) der person (2) unterhalb deren Kopfes (28) mindestens teilweise überdecken kann;

2. Vorrichtung nach Anspruch 1, dadurch gekennzeichnet, daß in dem mindestens einen Zwischenraum (5, 6, 7) eine Mehrzahl von Absaugströmen verlaufen kann

3. Vorrichtung nach Anspruch 1 bis 2, dadurch gekennzeichnet, daß der Schild (3) mit einer Mehrzahl von Abstandshaltern (10, 11, 12) versehen ist, die jeweils Abstände zueinander einhalten.

4. Vorrichtung nach Anspruch 1 bis 3, dadurch gekennzeichnet, daß die Abstandshalter (10, 11, 12) als im wesentlichen in gleichen Richtungen verlaufende Abstützleisten (13) ausgebildet sind.

5. Vorrichtung nach Anspruch 4, dadurch gekennzeichnet, daß der Schild (3) durch eine Mehrzahl von Abstützleisten (13) mit der Oberfläche (4) eine Mehrzahl von Zwischenräumen (5, 6, 7) ausbildet, die mit einem die Absaugströme erzeugenden Unterdruckerzeuger verbunden sind.

6. Vorrichtung nach Anspruch 4 und 5, dadurch gekennzeichnet, daß die Abstützleisten (13) sich im wesentlichen quer zur Körperlängsachse der Person (2) erstrecken.

7. Vorrichtung nach Anspruch 1 bis 6, dadurch gekennzeichnet, daß der Schild (3) aus einzelnen sich im wesentlichen quer zur Körperlängsachse der Person (2) erstreckenden Teilstücken (37) besteht, von denen jedes bezüglich dem anderen um Schwenkglieder (38) verschwenkbar angeordnet ist.

8. Vorrichtung nach Anspruch 4, 5 und 7, dadurch gekennzeichnet, daß die Abstützleisten (13) im wesentlichen in Richtung einer Körperlängsachse der Person (2) verlaufen.

9. Vorrichtung nach Anspruch 4 bis 8, dadurch gekennzeichnet, daß die Abstützleisten (13) einer Oberflächenform des Körperteils (1) angepaßt sind.

10. Vorrichtung nach Anspruch 1 bis 9. dadurch gekennzeichnet, daß die Abstandhalter (10, 11, 12) als Noppen ausgebildet sind.

11. Vorrichtung nach Anspruch 1 bis 10, dadurch gekennzeichnet, daß der Schild (3) als Brustschild (9) ausgebildet ist, der einen Oberkörper der Person (2) im Bereich der Brust und der vorderen Schulterpartie im wesentlichen überdecken kann.

12. Vorrichtung nach Anspruch 1 bis 14, dadurch gekennzeichnet, daß der Schild (3) an dem Rücken der Person (2) zugewandte Randbereiche aufweist, die an dem Rücken anliegen können.

13. Vorrichtung nach Anspruch 1 bis 9, dadurch gekennzeichnet, daß der Schild (3) die Achselhöhlen (14) der Person im wesentlichen umschließen kann.

14. Vorrichtung nach Anspruch 1 bis 13, dadurch gekennzeichnet, daß der Schild (3) einen Halsbereich (15) der Person (2) im Bereich ihrer Vorderseite mit einem Spalt (18) ausbildenden Abstand umgeben kann.

15. Vorrichtung nach Anspruch 1 bis 14, dadurch gekennzeichnet, daß der Schild (3) Bereiche der Oberarmgelenke (16) der Person (2) mit einem Abstand umgeben kann und so je einen Spalt (19, 20) ausbildet.

16. Vorrichtung nach Anspruch 1 bis 15, dadurch gekennzeichnet, daß der Schild (3) einen Bauchbereich (17) der Person (2) mit einem Abstand umgeben kann Und so je einen Spalt (21) ausbildet.

17. Vorrichtung nach Anspruch 1 bis 16, dadurch gekennzeichnet, daß die Spalten (18, 19, 20, 21) mit dem Zwischenraum (5, 6, 7) in Verbindung stehen und von den Absaugströmen durchströmbar sind.

18. Vorrichtung nach Anspruch 1 bis 17, dadurch gekennzeichnet, daß der Schild (3) über mindestens eine flexible Leitung (8) mit einem Unterdruckerzeuger verbunden ist.

19. Vorrichtung nach Anspruch 1 bis 18, dadurch gekennzeichnet, daß die Leitung (8) aus einem dem Bauchbereich (17) zugekehrten unteren Bereich des Schildes (3) an den Schild (3) herangeführt ist und in mindestens einem Bereich des Schildes (3) an dessen der Oberfläche (4) zugekehrten Seite mit mindestens einer Saugöffnung (24) versehen ist.

20. Vorrichtung nach Anspruch 18 und 19, dadurch gekennzeichnet, daß die Leitung (8) aus einem dem Halsbereich (15) zugekehrten oberen Bereich (26) des Schildes (3) an den Schild (3) herangeführt wird.

21. Vorrichtung nach Anspruch 18 bis 20, dadurch gekennzeichnet, daß die Leitung (8) als Zweigleitung ausgebildet ist und eine Mehrzahl von Zwischenräumen (5, 6) mit je einer Saugöffnung (24) versehen ist.

22. Vorrichtung nach Anspruch 21, dadurch gekennzeichnet, daß die Saugöffnungen (24) in einem Bereich des Schildes (3) angeordnet sind, der dem Halsbereich (15) nahe ist.

23. Vorrichtung nach Anspruch 21, dadurch gekennzeichnet, daß die Saugöffnungen (24) in einem zentralen Bereich des Schildes (3) angeordnet sind.

24. Vorrichtung nach Anspruch 21 bis 23, dadurch gekennzeichnet, daß die Saugöffnungen (24) auf den Halsbereich (15) der Person (2) ausgerichtet sind.

25. Vorrichtung nach Anspruch 1 bis 24, dadurch gekennzeichnet, daß der Schild (3) aus flexiblem Material besteht.

26. Vorrichtung nach Anspruch 1 bis 25, dadurch gekennzeichnet, daß der Schild (3) im wesentlichen aus einer Kunst- stoffolie besteht.

27. Vorrichtung nach Anspruch 1 bis 26, dadurch gekennzeichnet, daß der Schild (3) an seinem den Schultern der Person (2) zugekehrten Innenbereich eine schulterförmige Auflagefläche aufweist, mit der der Schild (3) über die Schultern (25) der Person (2) übergehakt werden kann.

28. Vorrichtung nach Anspruch 1 bis 27, dadurch gekennzeichnet, daß der Schild (3) mit einem Band verbunden ist, an dem er am Hals der Person (2) gehaltert werden kann.

29. Vorrichtung nach Anspruch 28, dadurch gekennzeichnet, daß das Band als die Schultern (25) der Person (2) überspannende und in deren Rückenbereich (40) geknüpfte Träger (39) ausgebildet ist.

30. Vorrichtung nach Anspruch 29, dadurch gekennzeicnet, daß die Träger (39) im Rückenbereich (40) kreuzweise angeordnet sind.

31. Vorrichtung nach Anspruch 29 und 30, dadurch gekennzeichnet, daß die Träger (39) im Rückenbereich (40) mit mindestens einem Band (41) verknüpft sind, das sich von einem dem Bauchbereich (17) zugewandten unteren Bereich des Schildes (3) in den Rückenbereich (40) erstreckt.

32. Vorrichtung nach Anspruch 33 bis 35, dadurch gekennzeichnet, daß die Leitung (8) entlang den Trägern (39) verläuft und im Halsbereich (15) in den Spalt (18) mündet.

33. Vorrichtung nach Anspruch 1 bis 27, dadurch gekennzeichnet, daß im Rückenbereich (40) der Person (2) mindestens ein den Schild (3) festlegender Verschluß vorgesehen ist, der zwischen einander zugekehrten und am Rücken der Person (2) anliegenden Randbereichen des Schildes (3) ange- ordnet ist.

34. Vorrichtung nach Anspruch 1 bis 33, dadurch gekennzeichnet, daß eine von der Körpertemperatur der Person (2) abhängige Regelung des von dem Unterdruckerzeuger erzeugten Luftdrucks vorgesehen ist.

35. Vorrichtung nach Anspruch 1 bis 34, dadurch gekennzeichnet, daß sie mit einer einen Kopf (28) der Person (2) im wesentlichen umschließbaren Kopfbewehrung versehen ist, die mit dem Unterdruckerzeuger verbunden ist.

36. Vorrichtung nach Anspruch 35, dadurch gekennzeichnet, daß die Kopfbewehrung aus einem Kopfschild (43) besteht, das lösbar mit dem Schild (3) verbunden ist.

37. Vorrichtung nach Anspruch 36, dadurch gekennzeichnet, daß der Kopfschild (43) den Kopf (28) der Person (2) überragen kann und so einen einen Rückenbereich (40) der Person (2) zugewandten Hinterkopf (62) unbedeckt läßt.

38. Vorrichtung nach Anspruch 36 und 37, dadurch gekennzeichnet, daß der Kopfschild (43) aus einer Halbschale besteht, die einen dem Hinterkopf (62) gegenüberliegenden Vorderteil des Kopfes (28) weiträumig umfassen kann.

39. Vorrichtung nach Anspruch 38, dadurch gekennzeichnet, daß die Halbschale beim Gebrauch dem Kopf (28) Bewegungsfreiheit lässt

40. Vorrichtung nach Anspruch 38 und 39, dadurch gekennzeichnet, daß die Halbschale durchsichtig ausgebildet ist .

41. Vorrichtung nach Anspruch 38 bis 40, dadurch gekennzeichnet, daß die Halbschale eine halbrunde Wölbung aufweist.

42. Vorrichtung nach Anspruch 38 bis 40, dadurch gekennzeichnet, daß die Halbschale eine sich vor einem Gesicht (45) der Person (2) erstreckende flache Ebene (46) aufweist, die in Richtung auf den Hinterkopf (62) verlaufende abgewinkelte Seitenflächen (56) aufweist.

43. Vorrichtung nach Anspruch 38 bis 42, dadurch gekennzeichnet, daß in der Halbschale ein Stirnanschlag (48) vorgesehen ist, der einer Stirn (47) der Person (2) gegenüber an einer dem Kopf (28) der Person (2) zugewandten Innenwandung der Halbschale befestigt ist und eine über die Länge einer Nase (49) der Person (2) hinausgehende Höhe aufweist.

44. Vorrichtung nach Anspruch 36 bis 43, dadurch gekennzeichnet, daß der Kopfschild (43) mit einem Rasterverschluß an den Schild (3) angekoppelt ist.

45. Vorrichtung nach Anspruch 44, dadurch gekennzeichnet, daß der Rasterverschluß als ein Klettverschluß (52) ausgebildet ist, dessen einer Teil (53) an einem bis in einen Brustbereich (51) der Person (2) ragenden Befestigungsansatz (50) des Kopfschildes (43) und dessen anderer Teil an einer im Brustbereich (51) der Person (52) vorgesehenen Verstärkung (54) des Schildes (3) vorgesehen ist.

46. Vorrichtung nach Anspruch 45, dadurch gekennzeichnet, daß die den Klettverschluß (52) tragende Verstärkung (54) an einer der Oberfläche (4) abgewandten Außenseite des Schildes (3) befestigt ist.

47. vorrichtung nach Anspruch 44, dadurch gekennzeichnet, daß der Kopfschild (43) mit Druckknöpfen an dem Schild (3) befestigt ist .

48. Vorrichtung nach Anspruch 44, dadurch gekennzeichnet, daß der Kopfschild (43) in einer am Schild (3) vorgesehenen Schiebehalterung mit einem Einschub befestigt ist.

49. Vorrichtung nach Anspruch 48, dadurch gekennzeichnet, daß der Einschub von einer dem Kopf (28) zugewandten Oberkante des Einschubes her in diesen eingesetzt ist.

50. Vorrichtung nach Anspruch 36 bis 49, dadurch gekennzeichnet, daß die Leitung (8) im Halsbereich (15) unter den Kopfschild (43) mündet.

51. Vorrichtung nach Anspruch 35 bis 50, dadurch gekennzeichnet, daß der Schild (3) und die Kopfbewehrung aus einem sterilisationsfähigen Material bestehen.

52. Vorrichtung nach Anspruch 35 bis 51, dadurch gekennzeichnet, daß der Schild (3) und die Kopfbewehrung von einem von der Person (2) getragenen Kittel (35) überspannbar sind.

**Claims**

1. Apparatus for the prevention of infections of a patient by germs which during an operation originate from a person (2) present at the operation, comprising a suction removal arrangement which can such the germs away from the region in which they originate on at least one part (1) of the body of the person (2) which is turned towards the patient during the operation, wherein the apparatus has a shield (3) which is substantially impermeable to air and the shield (3) is provided with at least one spacer (10, 11, 12) which is directed towards the body part (1) and which can be supported on the surface (4) of the body part (1) so that in use the shield (3) extends at a spacing relative to the surface (4) of the body part (1), with at least one intermediate space (5, 6, 7) between the shield and the surface, in which a suction removal flow which carries away the germs can pass, characterised in that the shield can at least partially cover over the body part (1) of the person (2) beneath the head (28) of the person.

2. Apparatus according to claim 1 characterised in that a plurality of suction removal flows can pass in the at least one intermediate space (5, 6, 7).

3. Apparatus according to claim 1 or claim 2 characterised in that the shield (3) is provided with a plurality of spacers (10, 11, 12) which are each at spacings from each other.

4. Apparatus according to claims 1 to 3 characterised in that the spacers (10, 11, 12) are in the form of support bars (13) which extend substantially in the same directions.

5. Apparatus according to claim 4 characterised in that the shield (3), by virtue of a plurality of support bars (13), forms with the surface (4) a plurality of intermediate spaces (5, 6, 7) which are in communication with a low-pressure generator for producing the suction removal flows.

6. Apparatus according to claim 4 and claim 5 characterized in that the support bars (13) extend substantially transversely to the longitudinal axis of the body of the person (2).

7. Apparatus according to claims 1 to 6 characterised in that the shield (3) comprises individual portions (37) which extend substantially transversely to the longitudinal axis of the body of the person (2) and each of which is arranged pivotably relative to the other about pivot members (38).

8. Apparatus according to claims 4, 5 and 7 characterised in that the support bars (13) extend substantially in the direction of a longitudinal axis of the body of the person (2).

9. Apparatus according to claims 4 to 8 characterised in that the support bars (13) are adapted to a surface shape of the body part (1).

10. Apparatus according to claims 1 to 9 characterised in that the spacers (10, 11, 12) are in the form of knobs.

11. Apparatus according to claims 1 to 10 characterised in that the shield (3) is in the form of breastplate which can substantially cover over an upper part of the body of the person (2) in the region of the chest and the front shoulder part.

12. Apparatus according to claims 1 to 14 characterised in that at the back of the person (2) the shield (3) has edge regions which are towards the back of the person and which can bear against the back of the person.

13. Apparatus according to claims 1 to 9 characterised in that the shield (3) can substantially enclose the armpits (14) of the person.

14. Apparatus according to claims 1 to 13 characterised in that the shield (3) can surround a neck region (15) of the person (2) in the region of the front side of the person at a spacing forming a gap (18).

15. Apparatus according to claims 1 to 14 characterised in that the shield (3) can surround regions of the upper arm joints (16) of the person (2) at a spacing and thus provides a respective gap (19, 20).

16. Apparatus according to claims 1 to 15 characterised in that the shield (3) can surround an abdominal region (17) of the person (2) at a spacing and thus provides a respective gap (21).

17. Apparatus according to claims 1 to 16 characterised in that the gaps (18, 19, 20, 21) communicate with the intermediate space (5, 6, 7) and can have the suction removal flows flowing therethrough.

18. Apparatus according to claims 1 to 17 characterised in that the shield (3) is connected to a low-pressure generator by way of at least one flexible line (8).

19. Apparatus according to claims 1 to 18 characterised in that the line (8) is taken to the shield (3) from a lower region of the shield (3) which is towards the abdominal region (17) and is provided with at least one suction opening (24) in at least one region of the shield (3) at its side which is towards the surface (4).

20. Apparatus according to claims 18 and 19 characterised in that the line (8) is taken to the shield (3) out of an upper region (26) of the shield (3), which is towards the neck region (15).

21. Apparatus according to claims 18 to 20 characterised in that the line (8) is in the form of a branch line and a plurality of intermediate spaces (5, 6) are each provided with a respective suction opening (24).

**22.** Apparatus according to claim 21 characterised in that the suction openings (24) are arranged in a region of the shield (30 which is near the neck region (15).

**23.** Apparatus according to claim 21 characterised in that the suction openings (24) are arranged in a central region of the shield (3).

**24.** Apparatus according to claims 21 to 23 characterised in that the suction openings (24) are oriented towards the neck region (15) of the person (2).

**25.** Apparatus according to claims 1 to 24 characterised in that the shield (3) comprises flexible material.

**26.** Apparatus according to claims 1 to 25 characterised in that the shield (3) essentially comprises a plastics foil.

**27.** Apparatus according to claims 1 to 26 characterised in that at its inner region which is towards the shoulders of the person (2), the shield (3) has a shoulder-shaped support surface with which the shield (3) can be hooked over the shoulders (25) of the person (2).

**28.** Apparatus according to claims 1 to 27 characterised in that the shield (3) is provided with a strap at which it can be held to the neck of the person (2).

**29.** Apparatus according to claim 28 characterised in that the strap is in the form of carriers (39) which extend over the shoulders (25) of the person (2) and connected in the back region (40) of the person.

**30.** Apparatus according to claim 29 characterised in that the carriers (39) are arranged crosswise in the back region (40).

**31.** Apparatus according to claims 29 and 30 characterised in that the carriers (39) are connected in the back region (40) to at least one strap (41) which extends into the back region (40) from a lower region of the shield (3), which is towards the abdominal region (17).

**32.** Apparatus according to claims 33 to 35 characterised in that the line (8) extends along the carriers (39) and opens into the gap (18) in the neck region (15).

**33.** Apparatus according to claims 1 to 27 characterised in that provided in the back region (40) of the person (2) is at least one closure for fixing the shield (3), which closure is arranged between mutually facing edge regions of the shield (3) which bear against the back of the person (2).

**34.** Apparatus according to claims 1 to 33 characterised in that there is provided a means dependent on the body temperature of the person (2) for regulating the air pressure generated by the low-pressure generator.

**35.** Apparatus according to claims 1 to 34 characterized in that it is provided with a head reinforcement for substantially enclosing a head (28) of the person (2) and which is connected to the low-pressure generator.

**36.** Apparatus according to claim 35 characterised in that the head reinforcement comprises a head shield (43) which is releasably connected to the shield (3).

**37.** Apparatus according to claim 36 characterised in that the head shield (43) can extend beyond the head (28) of the person (2) and thus leave uncovered a back part (62) of the head which is towards a back region (40) of the person (2).

**38.** Apparatus according to claims 36 and 37 characterised in that the head shield (43) comprises a half-shell portion which can extensively enclose a front part of the head (28), which is opposite to the rear part (62).

**39.** Apparatus according to claim 38 characterised in that the half-shell portion permits the head (28) freedom of movement in use.

**40.** Apparatus according to claims 38 and 39 characterised in that the half-shell portion is transparent.

**41.** Apparatus according to claims 38 to 40 characterised in that the half-shell portion has a half-round curvature.

**42.** Apparatus according to claims 38 to 40 characterised in that the half-shell portion has a flat plane (46) which extends in front of a face (45) of the person (2) and which has angled side surfaces (56) extending in the direction towards the rear part (62) of the head.

**43.** Apparatus according to claims 38 to 42 characterised in that provided in the half-shell portion is a forehead abutment (48) which is fixed opposite a forehead (47) of the person (2) to an inside wall of the half-shell portion which is towards the head (28) of the person (2), and is of a height which extends beyond the length of a nose (49) of the person (2).

44. Apparatus according to claims 36 to 43 characterised in that the head shield (43) is coupled to the shield (3) by a retaining closure.

45. Apparatus according to claim 44 characterised in that the retaining closure is in the form of a cling-type closure (52) of which one part (53) is provided on a fixing projection (50) of the head shield (43), which extends into a chest region (51) of the person (2), and the other part is provided on a reinforcement (54) of the shield (3), which is provided in the chest region (51) of the person (2).

46. Apparatus according to claim 45 characterised in that the reinforcement (54) which carries the cling-type closure (52) is fixed to an outside of the shield (3), which is remote from the surface (4).

47. Apparatus according to claim 44 characterised in that the head shield (43) is fixed to the shield (3) by press studs.

48. Apparatus according to claim 44 characterised in that the head shield (43) is fixed with an insert in a sliding holder provided on the shield (3).

49. Apparatus according to claim 48 characterised in that the insert is fitted thereinto from a top edge of the insert, which is towards the head (28).

50. Apparatus according to claism 36 to 49 characterised in that the line (8) opens in the neck region (15) under the head shield (43).

51. Apparatus according to claims 35 to 50 characterised in that the shield (3) and the head reinforcement comprise a sterilisable material.

52. Apparatus according to claims 35 to 51 characterised in that the shield (3) and the head reinforcement can be covered over by an overall (35) worn by the person (2).

**Revendications**

1. Dispositif pour éviter d'infecter un patient par des germes qui proviennent d'une personne (2) présente pendant une opération, comprenant une installation d'aspiration qui peut aspirer les germes de leur région d'origine sur au moins une partie (1) du corps de la personne qui est dirigée vers le patient pendant l'opération, le dispositif présentant un écran (3) sensiblement imperméable à l'air et l'écran (3) étant muni d'une entretoise (10,11,12) dirigée vers la partie (1) du corps, qui peut prendre appui sur la surface (4) de la partie (1) du corps de telle manière que l'écran (3) s'étende à un certain écartement de la surface (4) de la partie (1) du corps en laissant entre l'écran et cette surface au moins un espace intercalaire (5,6,7) dans lequel on peut faire circuler un courant d'aspiration qui évacue les germes, caractérisé en ce que l'écran peut recouvrir au moins Partiellement la partie (1) du corps de la personne (2) située au-dessous de sa tête (28).

2. Dispositif selon la revendication 1, caractértisé en ce qu'on peut faire circuler plusieurs courants d'aspiration dans cet au moins un espace intercalaire (5,6,7).

3. Dispositif selon l'une des revendications 1 à 2, caractérisé en ce l'écran (3) est muni de plusieurs entretoises (10,11,12) qui maintiennent des écartements.

4. Dispositif selon l'une des revendications 1 à 3, caractérisé en ce que les entretoises (10,11,12) sont constituées par des nervures d'appui (13) qui s'étendent toutes dans la même direction.

5. Dispositif selon la revendication 4, caractérisé en ce que l'écran (3) forme, avec la surface (4) plusieurs espaces intercalaires (5,6,7) qui sont reliés à un générateur de dépression produisant les courants d'aspiration.

6. Dispositif selon l'une des revendications 4 et 5, caractérisé en ce que les nervures d'appui (13) s'étendent sensiblement selon l'axe longitudinal du corps de la personne (2).

7. Dispositif selon l'une des revendications 1 à 6, caractérisé en ce que l'écran (3) est composé de Pièces partielles (37) qui s'étendent sensiblement transversalement à l'axe longitudinal du corps de la personne (2), dont chacune est disposée de manière à Pouvoir Pivoter par rapport à l'autre autour d'éléments d'articulation (38).

8. Dispositif selon l'une des revendications 4,5 et 7, caractérisé en ce que les nervures d'appui (13) s'étendent sensiblement parallèlement à l'axe longitudinal de la personne (2).

9. Dispositif selon l'une des revendications 4 à 8, caractérisé en ce que les nervures d'appui (13) sont adaptées à la forme de la surface de la partie (1) du corps.

10. Dispositif selon l'une des revendications 1 à 9, caractérisé en ce que les entretoises (10,11,12) sont constituées par des bossages.

11. Dispositif selon l'une des revendications 1 à 10, caractérisé en ce que l'écran (3) est constitué par un plastron (9) qui peut recouvrir sensiblement le torse de la personne (2) dans la région de la poitrine et de la partie avant des épaules.

12. Dispositif selon l'une des revendications 1 à 14, caractérisé en ce que l'écran (3) présente des régions marginales dirigées vers le dos de la personne et qui peuvent s'appliquer contre le dos.

13. Dispositif selon l'une des revendications 1 à 9, caractérisé en ce que l'écran (3) peut entourer sensiblement les aisselles (14) de la personne.

14. Dispositif selon l'une des revendications 1 à 13, caractérisé en ce que l'écran (3) peut entourer la région du cou (15) de la personne (2) dans la région de sa Partie avant, en laissant un écartement qui forme une fente (18).

15. Dispositif selon l'une des revendications 1 à 14, caractérisé en ce que l'écran (3) peut entourer des régions des articulations (16) des bras de la personne (2) à un certain écartement et forme ainsi une fente (19,20).

16. Dispositif selon l'une des revendications 1 à 15, caractérisé en ce que l'écran (3) peut entourer une région ventrale (17) de la personne (2) à un certain écartement et forme ainsi une fente.

17. Dispositif selon l'une des revendications 1 à 16, caractérisé en ce que les fentes (18,19,20,21) communiquent avec l'espace intercalaire (5,6,7) et peuvent être parcourues par les courants d'aspiration.

18. Dispositif selon l'une des revendications 1 à 17, caractérisé en ce que l'écran (3) est relié à un générateur de dépression par au moins une conduite flexible (8).

19. Dispositif selon l'une des revendications 1 à 18, caractérisé on ce que la conduite (8) s'étend d'une région inférieure à l'écran (3) dirigée vers la région centrale (17), le long de l'écran (3) et est munie, dans au moins une région de l'écran (3), sur le côté dirigé vers la surface (4), d'au moins une ouverture d'aspiration (24).

20. Dispositif selon l'une des revendications 18 et 19, caractérisé en ce que la conduite (8) s'étend le long de l'écran (3) en partant d'une région supérieure (26) de l'écran (3) dirigée vers la région du cou (15).

21. Dispositif selon l'une des revendications 18 à 20, caractérisé en ce que la conduite (8) est réalisée sous la forme d'une conduite ramifiée et plusieurs espaces intercalaires (5,6) sont munis chacun d'une ouverture d'aspiration (24).

22. Dispositif selon la revendication 21, caractérisé en ce que les ouvertures d'aspiration (24) sont disposées dans une région de l'écran (3) qui est proche de la région du cou (15).

23. Dispositif selon la revendication 21, caractérisé en ce que les ouvertures d'aspiration (24) sont disposées dans une région centrale de l'écran (3).

24. Dispositif selon l'une des revendications 21 à 23, caractérisé en ce que les ouvertures (24) sont dirigées vers la région du cou (15) de la personne (2).

25. Dispositif selon l'une des revendications 1 à 24, caractérisé en ce que l'écran (3) est fait d'une matière flexible.

26. Dispositif selon l'une des revendications 1 à 25, caractérisé en ce que l'écran (3) est essentiellement composé d'une feuille de matière Plastique.

27. Dispositif selon l'une des revendications 1 à 26, caractérisé en ce que l'écran (3) présente, dans sa région intérieure dirigée vers les épaules de la personne (2), une surface d'appui ayant la forme des épaules, avec laquelle l'écran (3) peut être accroché aux épaules (25) de la personne (2).

28. Dispositif selon l'une des revendications 1 à 27, caractérisé en ce que l'écran (3) est relié à une sangle par laquelle il peut être maintenu au cou de la personne (2).

**29.** Dispositif selon la revendication 28, caractérisé en ce que la sangle est constituée par une bretelle (39) qui passe sur les épaules (25) de la personne (2) et est nouée dans la région dorsale (40) de la personne.

**30.** Dispositif selon la revendication 29, caractérisé en ce que les bretelles (39) sont disposées en croix dans la région dorsale (40).

**31.** Dispositif selon l'une des revendications 29 et 30, caractérisé en ce que les bretelles (39) sont nouées dans la région dorsale (40) avec au moins une sangle (41) qui s'étend d'une région inférieure de l'écran (3) dirigée vers la région ventrale (17) Jusque dans la région dorsale (40).

**32.** Dispositif selon l'une des revendications 18 à 31, caractérisé en ce que la conduite (8) s'étend le long des bretelles (39) et débouche dans la fente (18) dans la région (15) du cou.

**33.** Dispositif selon l'une des revendications 1 à 27, caractérisé en ce que, dans la région dorsale (40) de la personne (2), est prévue au moins une fermeture qui immobilise l'écran (3), et qui est disposée entre les régions marginales de l'écran (3) qui sont dirigées l'une vers l'autre et sont appuyées contre le dos de la personne (2).

**34.** Dispositif selon l'une des revendications 1 à 33, caractérisé en ce qu'il est prévu une régulation de la pression d'air qui est produite par le générateur de dépression et qui est asservie à la température du corps de la personne (2).

**35.** Dispositif selon l'une des revendications 1 à 34, caractérisé en ce qu'il est muni d'une protection de tête qui entoure sensiblement la tête (28) de la personne (2) et qui est reliée au générateur de dépression.

**36.** Dispositif selon la revendication 35, caractérisé en ce que la protection de tête est constituée par un casque (43) qui est relié à l'écran (3) par des liaisons démontables.

**37.** Dispositif selon la revendication 36, caractérisé en ce que le casque (43) peut dépasser la tête (28) de la personne (2), et, ainsi, laisse découverte la partie arrière (62) de la tête qui est dirigée vers la région dorsale (40) de la personne (2).

**38.** Dispositif selon l'une des revendications 36 et 37, caractérisé en ce que le casque (43) est composé d'une demi-coquille qui peut entourer à large distance la partie avant de la tête (28) qui est à l'opposé de la partie arrière (62) de la tête.

**39.** Dispositif selon la revendication 38, caractérisé en ce que, en utilisation, la demi-coquille permet une liberté de mouvement de la tête (28).

**40.** Dispositif selon l'une des revendications 38 et 39, caractérisé en ce que la demi-coquille est transparente.

**41.** Dispositif selon l'une des revendications 38 à 40, caractérisé en ce que la demi-coquille présente un bombé semi-circulaire.

**42.** Dispositif selon l'une des revendications 38 à 40, caractérisé en ce que la demi-coquille présente un plan (46) plat qui s'étend devant le visage (45) de la personne (2), et qui présente des surfaces latéreles (46) coudées qui s'étendent en direction de la partie arrière (62) de la tête.

**43.** Dispositif selon l'une des revendications 38 à 42, caractérisé en ce que, dans la demi-coquille, est prévue une butée frontale (48) qui est fixée à la paroi intérieure de la demi-coquille dirigée vers la tête (28) de la personne (2) et présente une hauteur qui est supérieure à la longueur du nez (49) de la personne (2).

**44.** Dispositif selon l'une des revendications 36 à 43, caractérisé en ce que le casque (43) est accouplé à l'écran (3) au moyen d'une fermeture en encliquetage.

**45.** Dispositif selon la revendication 44, caractérisé en ce que la fermeture à encliquetage est constituée Par une fermeture (52) à glissière dont une partie (53) est prévue sur une partie de fixation (50) du casque (43) qui s'étend jusque dans la région (51) de la personne (2) et dont l'autre partie est prévue sur un prolongement (54) de l'écran (3) prévu dans la région (51) de la poitrine de la personne (52).

**46.** Dispositif selon la revendication 45, caractérisé en ce que le renforcement (54) portant la fermeture à glissière (52) est fixé à une face extérieure de l'écran (3) qui est à l'opposé de la surface (4).

**47.** Dispositif selon la revendication 44, caractérisé en ce que le casque (43) est fixé à l'écran (3) par des boutons-pression.

**48.** Dispositif selon la revendication 44, caractérisé en ce que le casque (43) est fixé au moyen d'une patte dans une coulisse prévue sur l'écran (3).

**49.** Dispositif selon la revendication 48, caractérisé en ce que la patte est engagée, à partir du bord supérieur de la patte qui est dirigée vers la tête (28) dans cette patte.

**50.** Dispositif selon l'une des revendications 36 à 49, caractérisé en ce que la conduite (8) débouche dans la région du cou (15) au-dessous du casque (43).

**51.** Dispositif selon l'une des revendications 35 à 50, caractérisé en ce que l'écran (3) et la protection de tête sont composés d'une matière stérilisable.

**52.** Dispositif selon l'une des revendications 35 à 51, caractérisé en ce que l'écran (3) et la protection de tête peuvent être recouverts pur une blouse (35) portée par la personne (2).

Fig. 1

Fig. 2

Fig. 3

Fig. 4

Fig. 6

Fig. 5